# EUROPEAN PATENT SPECIFICATION

(11) **EP 1 904 060 B1**
(45) Date of publication and mention of the grant of the patent: **01.06.2011**
(21) Application number: 06777523.9
(22) Date of filing: 29.06.2006
(51) Int. Cl.: A61K 31/409, A61K 33/08, A61K 33/24, A61K 33/30, A61P 31/04, A61P 35/00

(54) **USE OF PHTHALOCYANINE DERIVATES FOR THE NON-PHOTODYNAMIC TREATMENT OF DISEASES**
VERWENDUNG VON PHTHALOCYANIN-DERIVATEN ZUR NICHT-PHOTODYNAMISCHEN BEHANDLUNG VON ERKRANKUNGEN
UTILISATION DE DERIVES DE PHTALOCYANINE POUR LE TRAITEMENT NON PHOTODYNAMIQUE DE MALADIES

(30) Priority: 29.06.2005 US 695752 P; 04.04.2006 IT FI20060090
(43) Date of publication of application: 02.04.2008
(73) Proprietor: L. MOLTENI & C. DEI FRATELLI ALITTI SOCIETA' DI ESERCIZIO SOCIETA' PER AZIONI, 50018 Scandicci (Prov. of Florence) (IT)
(72) Inventor: RONCUCCI, Gabrio, I-53034 Colle Val d'Elsa (IT); FANTETTI, Lia, I-50139 Firenze (IT); CHITI, Giacomo, I-59012 Prato (IT); DEI, Donata, I-53037 San Gimignano (IT); ALONGI, Carmela, I-85037 San Brancato di Sant'Arcangelo (IT); COCCHI, Annalisa, I-59016 Poggio A Caiano (IT)
(74) Representative: Gervasi, Gemma
(86) International application number: PCT/EP2006/063698
(87) International publication number: WO 2007/000473

(56) References cited:
- EP-A- 0 906 758
- EP-A1- 1 164 135
- EP-A1- 1 356 813
- WO-A-98/33503
- JP-A- 9 059 279
- US-A- 5 281 616

## Description

### FIELD OF THE INVENTION

The invention relates to the field of phthalocyanine derivatives and in particular to the novel use of phthalocyanine derivatives of general formula (1), given hereinafter, for the preparation of pharmaceutical compositions useful in the non-photodynamic diagnosis and treatment of infections of various origin and disease characterised by cell hyperproliferation.

### STATE OF THE ART

Molecules containing the phthalocyanine chromofluorophore macrocycle have long been known to produce, by interaction with visible light, reactive oxygen species such as radicals or singlet oxygen, which are cytotoxic towards viruses, bacteria, fungi and eukaryotic cells.

Therefore, given also their agility to preferentially localise in pathologically affected areas, phthalocyanine compounds have been used for some time in so-called photodynamic therapy, commonly known as "PDT" therapy. PDT therapy consists of administering said compounds, allowing them to localise in the area to be treated, then irradiating said area with light in the visible region of the spectrum. Examples of compounds used in PDT therapy are described by Ben-Hur E. et al. in Int. J Radiat. Biol., Vol. 47, pp. 145-147, 1985. Other photosensitising agents useful in PDT are zinc phthalocyanine complexes and their conjugates described in US patent no. 5,965,598, in the name of the Applicant. These compounds have proved to be effective photosensitising agents in PDT treatment for both tumours and various infections. However, in many cases the pathologically affected parts involve internal organs, or in any event are not easily accessible to light.

EP 1 164 135 and EP 0 906 758 disclose compounds falling under the scope of the present general formula for use as sensitizer in PDT and diagnosis.

EP 1 356 813 discloses a photodynamic synergistic effect between phthalocyanines and chelating compounds like EDTA

JP 09 059279 discloses that a family of positively charged phthalocyanines containing quaternary ammonium groups has antimicrobial properties that are not associated with photodynamic activation. The central metal is either iron, cobalt, nickel or copper.

US 5 281 616 discloses the antimicrobial activity of a neutral phthalocyanine chelated to SiCl2.

From this the need arises to identify compounds able to manifest their cytotoxic action in darkness without having to irradiate the affected area with light.

### SUMMARY OF THE INVENTION

Contrary to the knowledge acquired to date in the state of the art and above illustrated with regard to the therapeutic applications of phthalocyanines, the Applicant has now found that the phthalocyanine derivatives of formula (I) given hereinafter, are also active in the dark. This obviously opens up previously inconceivable possibilities regarding the diagnostic and therapeutic use of said compounds, which could also be used in the internal regions of the human body that are inaccessible to light.

Therefore the present invention provides the use of the phthalocyanine derivatives of general formula (1) 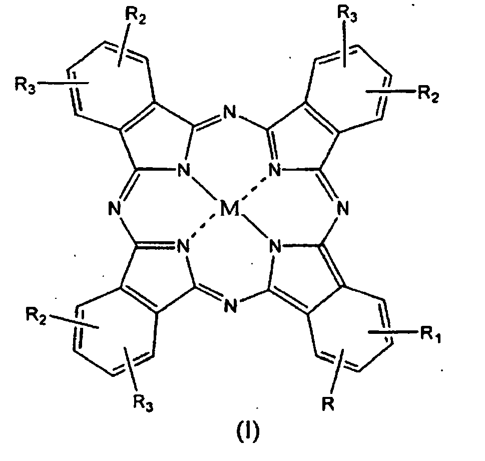 in which M is selected from 2H and a metal selected from the group consisting of Zn, Si(OR')₂. Ge(OR')₂ and AIOR', wherein R' is selected from H and alkyl groups having from 1 to 15 carbon atoms,
R is selected from H, groups comprising at least one quaternary ammonium substituent, groups comprising at least one aliphatic amino substituent, and groups suitable for conjugation to specific carriers,
R₁. equal or different from R, is selected from H, groups comprising at least one aliphatic amino substituent and groups comprising at least one quaternary ammonium substituent,
R₂ and R₃, equal or different from one another, are selected from H, alkoxy groups having from 1 to 10 carbon atoms, thioalkoxy groups having from 1 to 10 carbon atoms, groups comprising at least one aliphatic amino substituent and groups comprising at least one quaternary ammonium substituent,
with the proviso that:
a) at least one amongst R, R₁, R₂ and R₃ is a group comprising at least one aliphatic amino substituent or a group comprising at least one quaternary ammonium substituent and, when R, R₁, R₂ and R₃ are groups comprising at least one aliphatic amino substituent or groups comprising at least one quaternary ammonium substituent, or R and R₂ are groups comprising at least one aliphatic amino substituent or groups comprising at least one quaternary ammonium substituent and R₁ and R₃ are H, said groups comprising at least one aliphatic amino substituent or said groups comprising at least one quaternary ammonium substituent, are the same;
b) when R and R₁ are both different from H, they are in positions 1,4 or 2,3, whereas when only one between R and R₁ is different from H, it is in position 1 or 2;
c) when R₂ and R₃ are both different from H, they are in positions 8, 11, .15, 18, 22, 25 or 9, 10, 16, 17, 23, 24 whereas when only one between R₂ and R₃ is different from H, it is in positions 8(11), 15(18), 22(25) or in positions 9(10), 16(17), 23(24);
and their pharmaceutically acceptable salts,
for the preparation of pharmaceutical compositions useful in the non-photodynamic treatment of microbial infections and diagnosis and treatment of diseases characterised by cell hyperproliferation.

The characteristics and advantages of the invention will be illustrated in detail in the following description.

### DETAILED DESCRIPTION OF THE INVENTION

According to the invention, the expression "group comprising at least one quaternary ammonium substituent or one aliphatic amino substituent" means preferably a (X)ₚR₄ group, wherein X is selected from the group consisting of O, -CH₂-, CO, S, SO, and -NR₅ wherein R₅ is selected from H and C₁-C₁₅ alkyl; and R₄ is 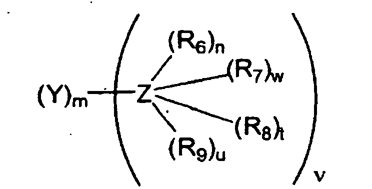 in which
Y is selected from the group consisting of C₁-C₁₀ alkyl and phenyl, possibly substituted, or Y forms with the Z group, to which it is bound, a saturated or unsaturated heterocycle, optionally substituted, which may comprise up to 2 heteroatoms selected from the group consisting of N, O and S;
Z is selected from the group consisting of -N, -CH₂N and -CONHCH₂CH₂N;
R₆ and R₇, equal or different from one another, are selected from the group consisting of C₁-C₁₅ alkyl and phenyl or form with the Z group, to which they are bound, a saturated or unsaturated heterocycle, optionally substituted, which may comprise up to two heteroatoms selected from the group consisting of N, O and S; R₈ and R₉, equal or different from each other, are selected from the group consisting of H, C₁-C₁₅ alkyl, and R₁₀COOEt or R₁₀COOMe groups in which R₁₀ is C₁-C₁₅ alkyl;
m, n, p, w, t and u, independently from one another, are 0 or 1; and v is an integer between 1 and 3,
with the proviso that only one amongst n, w, t and u is simultaneously 0.

According to the invention, of the groups comprising at least one aliphatic amino substituent, groups selected from the following are preferred:

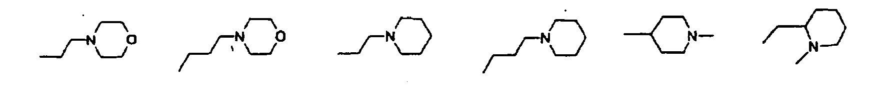

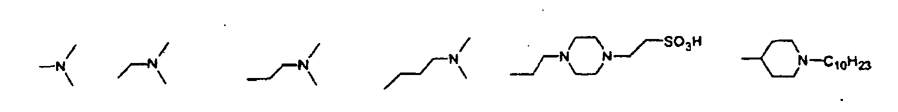

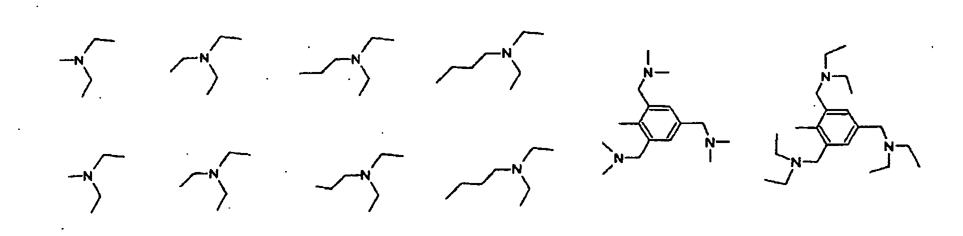

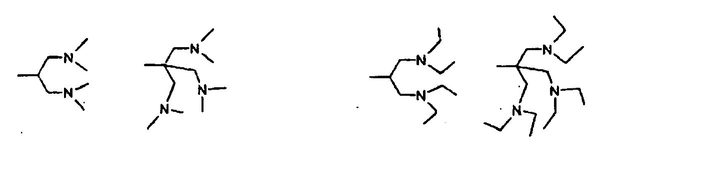

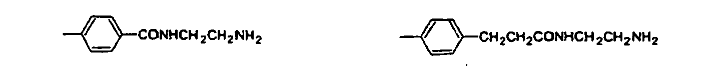

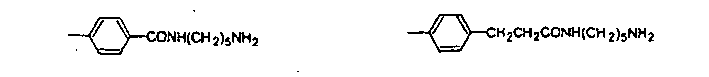

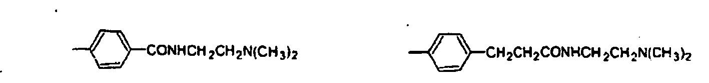

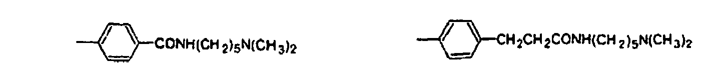

According to the invention, of the groups comprising at least one quaternary ammonium substituent, groups selected from the following are preferred:

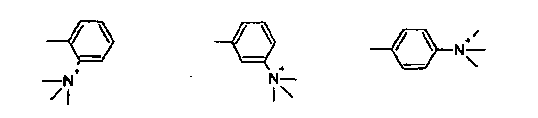

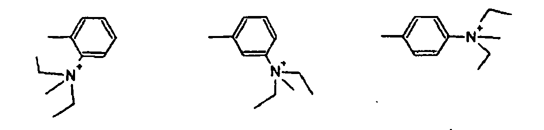

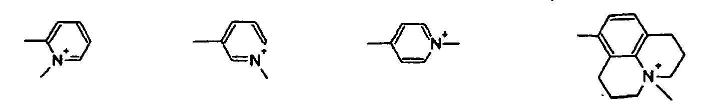

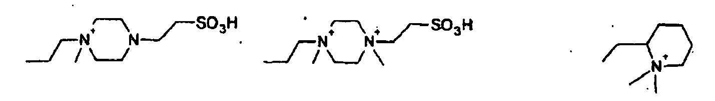

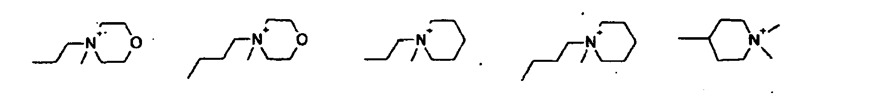

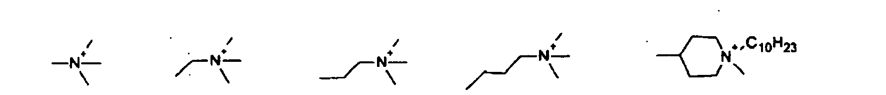

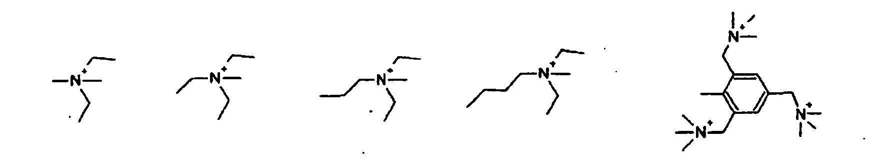

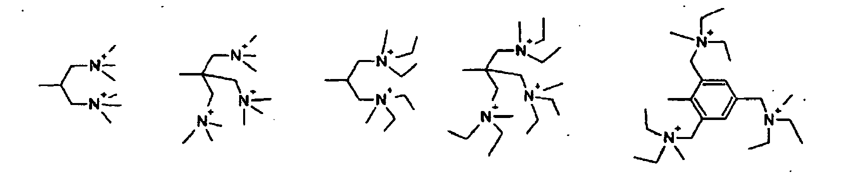

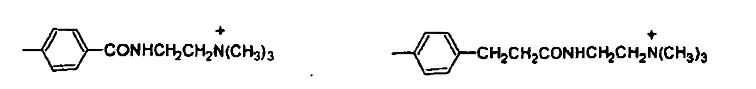

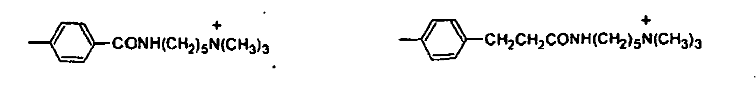

As groups comprising at least one aliphatic amino substituent, the following groups are particularly preferred:

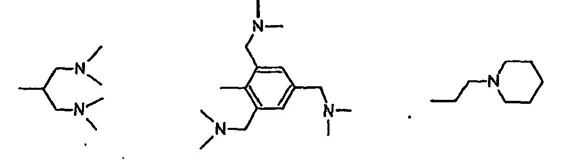

As groups comprising at least one quaternary ammonium substituent, the following groups are particularly preferred:

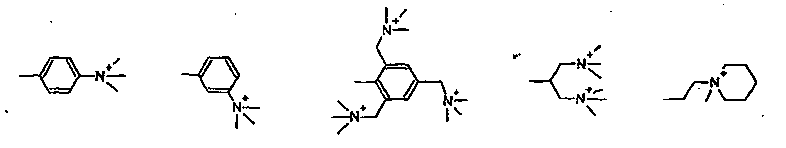

According to a preferred embodiment of the invention, M is Zn.

The expression "saturated or unsaturated heterocycle" means preferably a heterocycle selected from the group consisting of morpholine, piperidine, pyridine, pyrimidine, piperazine, pyrrolidine, pyrroline, imidazole, aniline and julolidine.

The expression "group suitable for conjugation to specific carriers" means any group suitable for covalently binding to bio-organic carriers chosen in the group comprising of amino acids, polypeptides, proteins, antibodies, polysaccharides and aptamers; able to facilitate transport of the phthalocyanines to particular targets; the aforesaid expression preferably indicates a group selected from the group consisting of -COOH, -SH, -NH₂, -CO-CH₂-Br, -SO₂Cl, maleimide, hydrazine, phenol, imidate, biotin, optionally bound to the phthalocyanine nucleus through a suitable spacer (X)ₚ-W, where X and p are defined as above and W is selected from C₁-C₁₀ alkyl, aryl, and C₁-C₅ arylalkyl.

When R is a group suitable-for conjugation to specific carriers, as defined above, R₁ is preferably equal to H and R₂ and R₃ are selected from H, groups comprising at least one aliphatic amino substituent, and groups comprising at least one quaternary ammonium substituent, provided that at least one of R₂ and R₃ is different from H.

The phthalocyanine derivatives of formula (I) can be prepared starting from the corresponding amino derivatives, which in their turn can be prepared from commercially available products by known procedures, such as those described in US Patent No. 5,965,598, in European Patent No. 1164 135 and in European Patent No. 1381 611, all in the name of the Applicant.

Preferred are:
- di- and mono-substituted compounds of formula (I), i.e. compounds in which: R is selected from groups comprising at least one quaternary ammonium substituent and groups comprising at least one aliphatic amino substituent, as defined above, R₁ = R or R₁ is H, and R₂ = R₃ = H;
- the compounds of formula (I) tetra-substituted with the same substituents, i.e. the compounds in which the R and R₂ substituents are the same and selected from groups comprising at least one quaternary ammonium substituent and groups comprising at least one aliphatic amino substituent defined as above, and R₁ = R₃ = H; and
- the tetra-substituted compounds of formula (I) suitable for conjugation, in which R is selected from groups suitable for conjugation to specific carriers, as defined above, R₂ is selected from groups comprising at least one quaternary ammonium substituent and groups comprising at least one aliphatic amino substituent, and R₁ =R₃=H._{.}

Optimal results in terms of non-photodynamic activity have been observed for the aforesaid compounds of formula (I) tetra-substituted with the same substituents, and for the di- and mono-substituted compounds, but in particular for the di- and mono-substituted compounds.

For the purposes of the present invention, the expression "non-photodynamic activity" means cytotoxic activity in the absence of light radiation, i.e. in darkness; while the expression "diseases characterised by cell hyperproliferation" means for example tumours and pre-cancerous and proliferative pathologies, such as psoriasis, actinic keratosis, atheroma, endoarterial hyperplasia and prostatic hyperplasia.

The compounds of formula (I) have proven to be particularly useful in the treatment of microbial infections caused by Gram- and Gram+ bacteria, and fungi, and preferably Gram+ bacteria and fungi.

The best results have been observed with fungal infections.

According to the invention, the present compounds of formula (I) are used for the preparation of pharmaceutical compositions as single active principles or in combination with one or more further active principles and/or in combination with a metal chelating agent; such pharmaceutical compositions may also comprise excipients and pharmaceutically acceptable diluents.

Particularly preferred are the pharmaceutical compositions of the invention comprising, in addition to a compound of formula (I), a metal chelating agent preferably selected from metal chelating agents having specificity for Ca²⁺ and Mg²⁺ ions, such as 1,2-diaminocyclohexane-N,N,N',N'-tetraacetic acid (CDTA), diethylenetriaminepentaacetic acid (DTPA), ethylenediamine-N,N,N',N'-tetraacetic acid (EDTA), citric acid and its salts. Compositions comprising EDTA are particularly preferred.

The present compositions are preferably formulated as aqueous solutions, creams, gels, ointments, liposomal formulations, tablets, capsules, suspensions etc., although formulations other than the aforestated are be considered as included within the scope of the invention. Topical administration is the preferred route, but the present compositions can also be used for parenteral, oral, nasal administration, etc.

In staid pharmaceutical composition, the dosages of active principles can vary for example between 0.1 and 20 mg of the product of formula (I) per Kg body weight, preferably ranging between 0.2 and 5 mg per Kg body weight.

The following examples of the present invention are given by way of illustration.

### EXAMPLE 1

### Preparation of the compound of formula (I) in which M is Zn_{.} R₁=R₂=R₃=H and R = {2-(trimethylammonium)-1-[(trimethylammonium) methyl]ethoxy} dichloride in position 2 [Compound 1]

### a) Synthesis of 2-{2-dimethylamino-1- methyl]ethoxy} zinc phthalocyaninate

Under nitrogen atmosphere 48 g of 4-{2-(dimethytamino)-1-[(dimethylamino) methyl]ethoxy} phthalonitrile (0.18 mol) and 68 g of 1,2-dicyanobenzene (0.53 mol) are dissolved in 420 ml of DMF.

32.4 g of Zn(AcO)₂ (0.18 mol) and 136 ml of DBU (0.90 mol) are added and the reaction mixture is brought to 130°C then maintained at this temperature for 20 hours, shielded from light, under nitrogen atmosphere and under vigorous stirring.

The reaction mixture is then cooled to about 50°C and treated with 800 ml of deionised H₂O; the suspension thus obtained is filtered and the recovered solid washed with H₂O in portions (2 x 400 ml) and with 8/2 acetonelethyl ether (2 x 500 ml).

The product is subjected to chromatographic purification on silica gel (eluent: 9/1 THF/DMF), followed by re-precipitation from DMF (400 ml)/Et₂O (1.6 I)/n-hexane (12 l) to obtain 35.6 g of product (yield = 27%).

¹H-NMR (300 MHz, DMSO-d₆): δ = 9.28-9.25 (m, 6H), 9.14 (d, 1H, J = 8.4 Hz), 8.87(s, 1H), 8.21-8.19 (m, 6H), 7.79 (d, 1H, J = 8.4Hz), 5.17 (t, 1H, J = 5 Hz) 2.86 (d, 4H, J = 5 Hz), 2.5 (s, 12H) ppm.

ESI-MS: m/z 721 [(M+H)⁺].

### b) Synthesis of 2-{2-(trimethylammonium)-1-[(trimethylammonium) methyl]ethoxy} zinc phthalocyaninate diiodide

30.2 g (0.042 mol) of 2-{2-(dimethylmino)-1-[(dimethylamino) methyl]ethoxy} Zinc phthalocyaninate, obtained as described above in point a), are dissolved in 900 ml of NMP. 60 ml of Mel (1 mol) are added and the solution maintained at room temperature for 72 hours, shielded from light, under nitrogen atmosphere and under vigorous stirring. The reaction mixture is diluted with 1.4 l of MeOH, then treated with 5.5 l of ethyl ether. The suspension thus obtained is left under stirring for 30 minutes, allowed to stand for 1 hour then filtered. The recovered solid is washed with ethyl ether (4 x 0.5 l) to finally obtain 47 g of wet product, found to be 2-{2-(trimethylammonium)[(trimethylammonium) methyl]ethoxy} zinc phthalocyaninate diiodide.

¹H-NMR (300 MHz, DMSO-d₆): δ = 9.46-9.43 (m, 7 H), 9.19 (bs = 1H), 8.29-8.27 (m, 6H), 8.17 (d, 1H, J = 10 Hz), 6.20-6.10 (m, 1H), 4.22-4.04 (m = 4H), 3.41 (s, 18H) ppm.

### c) Synthesis of 2-{2-(trimethylammonium)-1-[(trimethylammonium) methyl]ethoxy} zinc phthalocyaninate dichloride

10.1 g of this product are dissolved in 250 ml of a 4/1 MeOH/DMF mixture. The solution is passed through a chromatography column having 500 g of Amberlite^{®} IRA 400 (CI) resin as the stationary phase, previously washed with an aqueous solution made acid with 0.5 M HCl and conditioned with 4/1 MeOH/DMF. Ethyl ether (2 1) is slowly added to the eluate (about 500 ml) while maintaining under stirring, and the obtained suspension is allowed to rest for 1 hour at 4°C then filtered; the recovered solid is washed with ethyl ether (4 x 500 ml) to obtain, after drying, 6.9 g of the title product.

1H-NMR (300 MHz, DMSO-d₆): δ = 9.43-9.40 (m, 7 H), 9.19 (bs = 1H), 8.26-8.22 (m, 6H), 8.17 (d, 1H, J = 10 Hz), 6.20-6.18 (m, 1H), 4.19-4.17 (m = 4H), 3.42 (s, 18H) ppm.

¹³ C-NMR (75 MHz, DMSO-d₆): δ = 156.20, 154.40, 154.17, 153.95, 153.63, 153.08, 141.07, 139.09, 138.93, 134.42, 130.18, 124.72, 123.15, 120.31, 111.04, 69.36, 67.42, 54.57 ppm.

UV-vis (DMF) λₘₐₓ(%): 672 (100), 606 (9), 341(11).

ESI-MS: m/z 375 [(M-2Cl)²⁺¹

By using the procedure previously described in example 1, the following phthalocyanine derivatives of formula (I) were also prepared:

### EXAMPLE 2

Compound of general formula (I) in which M is Zn, R₁=R₂=R₃=H and R= {2-(dimethylamino)-1-[(dimethylamino) methyl]ethoxy} in position 2 [Compound 2]

### EXAMPLE 3

Compound of general formula (I) in which M is Zn, R₁=R₂=R₃=H and R= {2-(trimethylammonium)-1-[(trimethylammonium) methyl]ethoxy} diiodide in position 1 [Compound 3]

### EXAMPLE 4

Compound of general formula (I) in which M is Zn, R₁=R₂=R₃=H and R= [2-(*N-*methyl-1-piperidinium) ethoxy] iodide in position 1 [Compound 4]

### EXAMPLE 5

Compound of general formula (I) in which M is Zn, R₁=R₂=R₃=H and R= [3-(*N,N,N*-trimethylammonium) phenoxy] iodide in position 2 [Compound 5]

### EXAMPLE 6

Compound of general formula (I) in which M is Zn, R₁=R₂=R₃=H and R= [4-(*N,N,N* trimethylammonium) phenoxy] iodide in position 1 [Compound 6]

### EXAMPLE 7

Compound of general formula (I) in which M is Zn, R₁=R₂=R₃=H and R= (*N-*methylpyridinium-3-oxy) iodide in position 1 [Compound 7]

### EXAMPLE 8

Compound of general formula (I) in which M is Zn, R₁=R₂=R₃=H and R= {2,4,6-tris-[(*N,N*dimethylamino) methyl]phenoxy} in position 2 [Compound 8]

### EXAMPLE 9.

Compound of general formula (I) in which M is Zn, R₁=R₂=R_{J}=H and R= (2,4,6-tris-[(*N,N,N-*trimethylammonium) methyl]phenoxy) trichloride in position 2 [Compound 9]

### EXAMPLE 10

### Preparation of the compound of general formula (I) in which M is Zn, R₂=R₃- =H, R=R₁= {[3-(dimethylamino)phenyl]thio}, with R and R₁ in positions 2 and 3 [Compound 10]

### a) Synthesis of 2.3-bis{[3-(dimethylamino)phenyl]thio} zinc phthalocyaninate

DBU (1.22 g. 7 mmol) was added to a solution of 4,5-bis{[3-(dimethylamino)phenyl]thio}phthalonitrile (107 mg, 0.2 mmol), 1,2-dicyanobenzene (77 mg, 0.6 mmol) and Zn(OAc)₂ (183 mg, 0.8 mmol) in anhydrous DMF (6 ml). The thus obtained mixture was heated to 120°C and maintained under stirring for 3 hours under inert atmosphere and shielded from light, returned to room temperature then poured into 50 ml of deionised water. The blue precipitate thus obtained was separated by centrifugation then subjected to two chromatographic passages on silica gel (flash (THF) and MPLC (3/2 n-hexane/THF)) to give 33 mg of the desired product (yield = 19%).

¹H-NMR (300 MHz, *d*₆-DMSO): δ 8.98-8.96 and 8.81-8.73 (8H, two m), 8.12-8.07 (6H, m), 7.46 (1H, dd, J₁=J₂=8.4 Hz), 7.06-7.04 (2H, m), 6.93 (1H, d, J=9.6 Hz), 2.99 (12H, s).

UV-vis (DMF): nm (%)685 (100), 345 (28.1), 613 (17.3).

FAB⁺-MS: m/z 880 [(C₄₈ H₃₄ N₁₀ S₂ Zn)+H]⁺

### b) Synthesis of 2,3-bis{[3-(trimethylammonium)phenyl]thio} zinc phthalocyaninate diiodide

1 ml of iodomethane was added to a solution of Zinc 2,3-bis{[3-(dimethylamino)phenyl]thio)-phthalocyaninate (50 mg, 0.06 mmol) in 3 ml of anhydrous NMP. The solution was maintained at room temperature for 8 hours, under stirring and shielded from light, then treated with ethyl ether. The blue precipitate thus obtained was separated from the mother liquors, redissolved in methanol and reprecipitated from ethyl ether. 44 mg of the desired product were obtained (yield = 85%).

¹H-NMR (300MHz, *d*₆-DMSO): δ ppm. 9.45-9.41 (4H, m); 9.34 (2H, s), 9.25-9.22 (2H, m), 8.46 (2H, bs), 8.35-8.27 (6H, m), 8.13-8.09 (1H, m), 7.88-7.74 (6H, m), 3.35 (18H, s).

¹³C-NMR (75 MHz, DMSO-*d*₆) δ154.40, 154.04, 152.78, 150.22, 149.10, 138.58, 138.38. 138.27, 138.81, 136.32, 1 32.23, 131.95. 130.53, 130.29, 130.20, 126.85, 123.27, 123.12, 122.82, 120.38, 57.26. UV-vis (DMF): nm (%) 690 (100), 334 (34.6), 613 (17.0), λ₆₉₀ = 126000 M⁻¹cm⁻1b.

ESI-MS: m/z 454 [(C₅₀ H₄₀ N₁₀ S₂ Zn)-2I+2H]²⁺

By using the procedure described above in Example 10, the following compound was also prepared:

### EXAMPLE 11

Compound of general formula (I) in which M is Zn, R₂=R₃=H, R=R₁= {3-(*N,N,N-*trimethylammonium)phenoxy] iodide, with R and R₁ in positions 2 and 3 [Compound 11]

### EXAMPLE 12

### Preparation of compound of general formula (I) in which M is Zn, R₁=R₃=H, R=R₂= (3-(N,N,N-trimethylammonium)phenoxyl tetraiodide, with R and R₂ in positions 2,9(10),16(17),23(24) [Compound 12]

### a) Synthesis of 2,9(10),16(17), 23(24)-tetra[3-(N,N-dimethylamino)phenoxyl Zinc phthalocyaninate

DBU (29 ml -194 mmol) and anhydrous Zn(OAc)₂ (3.48 g - 19 mmol) were added to 3-(*N,N*-dimethylamino)phenoxy] phthalonitrile (10 g - 38 mmol); the mixture thus obtained was brought to 160°C and maintained at this temperature for 4 hours, under stirring, under inert atmosphere and shielded from light. After having returned the mixture to room temperature, it was treated with 200 ml of deionised water and the solid obtained separated and washed with water and methanol. The crude product was then subjected to chromatographic purification (silica gel, CH₂CI₂/MeOH 98/2 v/v). The eluate containing the desired compound as a mixture of positional isomers was concentrated dissolved in CH₂Cl₂ and reprecipitated from n-hexane to give 7.62 g of pure isomeric mixture (yield = 72%). UV-Vis (DMF) λₘₐₓ (nm) 681 (ε=70300 M⁻¹ cm⁻¹) 612, 356

¹H-NMR (200 MHz, DMSO-*d*₆) δppm 9.01-8.90 (m, 4H), 8.51-8.45 (m, 4H), 7.82-7.73 (m, 4H), 7.49-7.36 (m, 4H), 6.85-6.73(m, 12H), 3.05-3.02 (m, 24H).

¹³C-NMR (300 MHz, DMSO-*d*₆) δ ppm 159.71, 159.47, 158.33, 158.21, 153.06, 152.53, 152.23, 152.03, 151.77, 151.36, 139.91, 132.89, 131.16, 131.02, 124.23, 120.32. 110.76, 109.17, 107.97, 107.83, 104.59

FAB-MS *m*/*z* 1117 [M+H]⁺.

### b) Synthesis of 2,9(10),16(17),23(24)-tetra[3-(N,N,N-methylammonium)phenoxy] zinc phthatocyaninate tetraiodide

An excess of iodomethane (16 ml) was added to a solution of 2,9(10),16(17),23(24)-tetra[3-(*N,N*-dimethylamino)phenoxy] Zinc phthalocyaninate (6.32 g - 5.65 mmol) in NMP. (158 ml) and the mixture maintained under stirring for 120 hours, at room temperature and shielded from light, then diluted with methanol (320 ml) and treated with ethyl ether (1.3 1) to give a green precipitate corresponding to the desired product in the form of an isomeric mixture (9 g, 95% yield).

UV-Vis (DMF) λₘₐₓ(nm) 677 (ε =161000 M⁻¹ cm⁻¹), 609, 353;

¹H-NMR (200MHz, DMSO*-d*₆) δ ppm 9.55-9.43 (m, 4H), 9.09-9.02 (m, 4H), 8.22-8.15 (m, 4H), 8.07-7.76 (m, 12H), 7.62-7.52 (m, 4H) 3.77 and 3.75 (2s, 36H)

¹³ C-NMR (200 MHz, DMSO*-d*₆) δ ppm 157.84, 157.67, 152.50 (m), 148.85, 140.00 (m), 134.00, 131,77, 124.70, 121.30 (m), 120.18. 119.89, 115.99, 115.80, 112.70, 112.42, 56.60

ESI-MS *m*/*z* 388 [M -41 -CH3]³⁺, 573 [M -41 -2CH3]²⁺ 1132 [M -41-3CH3]⁺.

By using the procedure previously described in Example 12 and in Example 1c), the following phthalocyanine derivatives of formula (I) were also prepared:

### EXAMPLE 13

Compound of general formula (I) in which M is Zn, R₁=R₃=H, R=R₂= [3-(*N,N,N-*trimethylammonium)phenoxy] chloride, with R and R₂ in positions 1, 8(11), 15(18), 22(25) [Compound 13]

### EXAMPLE 14

Compound of general formula (I) in which M is Zn, R₁₌R₃=H, R=R₂= [4-(*N,N,N-*trimethylammonium)phenoxy] chloride, with R and R₂ in positions 1, 8(11), 15(18), 22(25) [Compound 14]

### EXAMPLE 15

Compound of general formula (I) in which M is Zn, R=R₁=R₂= R₃= [3-(*N,N,N-*trimethylammonium)phenoxy] chloride, with R, R₁, R₂, R₃ in positions 2,3,9,10,16,17,23,24 [Compound 15]

### EXAMPLE 16

### Evaluation of antimicrobial activity in darkness

### a) In vitro experiments

An evaluation of *in vitro* non-photodynamic antimicrobial activity was carried out for Compounds 1-12 prepared as described above, by using the following procedure.

Cells of *Candida albicans* (ATCC 10231) were grown in Fluid Sabouraud Medium (Difco Laboratories, Detroit, MI) at 37°C under aerobic conditions. Cells in the stationary growth phase were harvested (3,000 g, 15 min), washed with phosphate buffer saline (PBS), and diluted to a final concentration of 1x10⁶ cells/ml corresponding to an absorbance of 0.12 at 630 nm.

Samples (1 ml) were prepared by adding to PBS suitable volumes of the phthalocyanine stock solutions in DMSO (the final concentrations of the tested phthalocyanines range from 1 to 50 µM) so that the final amount of DMSO in the sample is 5% v/v. The samples were then incubate 37°C for 1 h. Cell survival was determined by serial 10-fold dilution in PBS of sample aliquots (10 µl), then plated in triplicate onto Sabouraud dextrose agar (Difco). After incubation of the plates at 37°C for 24 h, colony forming units (CFU/ml) were counted and the minimal bactericidal concentration (MBC), corresponding to the concentration of active principle that cause a ≥ 4 log₁₀ of reduction of CFU/ml) was determined.

Control experiments were carried out in the absence of the phthalocyanine derivative. All experiments were repeated in triplicate.

The MBC values of the tested compounds, as well as that ones found with the same protocol for two known antibiotics, nystatin and amphotericin, are reported in the following Table 1.

**Table 1**

| **Compound** | **MBC (µm)** |
|---|---|
| 1 | 5 |
| 2 | 5 |
| 3 | 1 |
| 4 | 10 |
| 5 | 10 |
| 6 | 10 |
| 7 | 10 |
| 8 | 10 |
| 9 | 10 |
| 10 | 5 |
| 11 | 10 |
| 12 | 10 |
| Nystatin | 10 |
| Amphotericin | 10 |

From the results in the Table 1 above, it is evident that the present phthalocyanine derivatives have a non-photodynamic antimicrobial activity equivalent to that of the two antibiotics taken as a reference, that are the most used antifungal antibiotics due to their remarkable fungicidal activity. Compounds 1-3 and 10 have shown MBC values even higher than those of nystatin and amphotericin.

### b) In vivo experiments

An animal model of infection (*S. aureus* wound infection in guinea-pig) was used to assess the *in vivo* activity of Compounds 1, 9, 13, 14, and 15 prepared as described above, in comparison to classic antibiotic regimen for the treatment of infections caused by Gram+ bacteria, consisting in administration of Imipenem, the most used and effective antimicrobial agent for this kind of infections.

The experimental protocol is reported below.

Animals: Dunkin-Hartley guinea-pigs (weight, 450 to 500 g) were used throughout the study.

Bacteria: *Staphylococcus aureus* (ATCC 6538) was used. Bacterial colonies were harvested after overnight growth on TSA (Tryptic Soy Agar) and suspended in phosphate-buffered saline (PBS, pH=7.4) to achieve a standard turbidity (A₆ₒₒ reading of 0.4). Serial 10-fold and 2-fold dilutions were performed to prepare a range of inocula. The inoculum of 2 x10⁷ cells/ml was mixed 1:1 (vol/vol) ratio with dextran microbeads (Cytodex, Sigma Chemicals Co., St.Louis, Mo) and used in our experiments. Backcounts were determined in triplicate and averaged to determine precise size of the bacterial inoculum.

Guinea pig model: The dorsal hair of animals was removed and a grid was drawn designating 8 areas for intermuscular inoculation. Each site of 2 guinea pigs for treatment groups was inoculated with 0.2 ml of the bacterium-bead suspensions. Control guinea pigs received PBS (vehicle). After the inoculation the guinea pigs were returned to their cages. After one day, 0.1 ml of solutions in PBS of the above said selected Compounds of the invention, or of lmipenem was administered in each abscess.

One day later, the guinea pigs were sacrificed; by using a sterile technique abscess were removed, weighed and homogenised in 3 ml of saline solution for 2 min (Ultraturrax). Homogenates were diluted and plated onto blood agar. The plates were incubated at 37°C for 24 h and the number of colony forming units (CFU/ml) was recorded. The bacterial count means of the compound-treated groups were compared to the vehicle-treated group to give percentage inhibition of abscess development. The abscess weight means of the compound-treated groups were compared to the vehicle-treated group to give percentage abscess decreasing. All experiments were repeated two times in different days.

The obtained results are summarised in the following Tables 2-7, related to Compounds 1, 9, 13, 14, 15 and to imipenem respectively.

**Table 2 - Compound 1**

| **Concentration (µg/kg)** | **CFU inhibition %** | **Abscess decreasing %** |
|---|---|---|
| 0.08 | 0 | 0 |
| 0.16 | 48.2±2.4 | 11.6 ± 0.5 |
| 0.8 | 54.7 ± 8.1 | 11.5 ± 1.9 |
| 1.6 | 69.0 ± 6.7 | 23.0 ± 3.9 |
| 16 | 71.5 ± 7.7 | 20.1 ±5.2 |
| 160 | 89.4±3.8 | 25.6±4.3 |

**Table 3 - Compound 9**

| **Concentration (µg/kg)** | **CFU inhibition %** | **Abscess decreasing %** |
|---|---|---|
| 0.002 | 0 | 0 |
| 0.1 | 4.8 ± 3.0 | 0 |
| 0.2 | 45.3 ± 1.2 | 1 0.6 ± 0.8 |
| 1 | 55.1 ± 6.6 | 9.9 ± 2.4 |
| 2 | 55.9 ± 7.2 | 11.7 ± 1.3 |
| 20 | 80.0 ± 8.1 | 30.6 ± 12.0 - |
| 200 | 94.5 ± 5.2 | 30.9 ± 5.7 |

**Table 4 - Compound 13**

| **Concentration (µg/kg)** | **CFU inhibition %** | **Abscess decreasing %** |
|---|---|---|
| 0.044 | 0.0 | 0.0 |
| 0.22 | 56.4 ±7.3 | 16.6 ± 3.8 |
| 0.44 | 57.8 ± 9.6 | 16.8 ± 6.3 |
| 4.4 | 73.2 ± 9.8 | 20.3 ± 5.5 |
| 44 | 64.6 ± 11 | 20.1 ± 6.2 |
| 440 | 80.4 ± 9.4 | 25.3 ± 8.7 |

**Table 5 - Compound 14**

| **concentration (µg/kg)** | **CFU inhibition %** | **Abscess decreasing %** |
|---|---|---|
| 0.44 | 39.7 ± 8.1 | 9.5 ± 4.3 |
| 4.4 | 10.0 ± 5.5 | 1.5 ± 1.0 |
| 44 | 57.9 ± 8.1 | 9.8 ± 4.1 |
| 440 | 80.0 ± 9.5 | 22.8 ± 4.2 |

**Table 6 - Compound 15**

| **Concentration (µg/kg)** | **CFU inhibition %** | **Abscess decreasing %** |
|---|---|---|
| 0.41 | 5.7 ± 1.5 | 0 |
| 2.05 | 5.5 ± 3.1 | 0 |
| 4.1 | 38.8 ± 1.9 | 1.9 ± 0.5 |
| 41 | 59.4 ± 9.6 | 20.6 ± 7.2 |
| 410 | 62.2 ± 9.0 | 21.8 ± 6.2 |

**Table 7 - Imipenem**

| **concentration (µg/kg)** | **CFU inhibition %** | **Abscess decreasing %** |
|---|---|---|
| 83200 | 77.0113.5 | 3.6 ± 1.0 |
| *Total dose of 1152 mg*/*kg in 3 days* (384 mg*/*kg*/*day)* | 64.4 ± 7.4 | n.d. |

| | | |
|---|---|---|
| *Classic regimen for parenteral administration | | |

## Claims

1. Use of phthalocyanines derivatives of general formula (I) 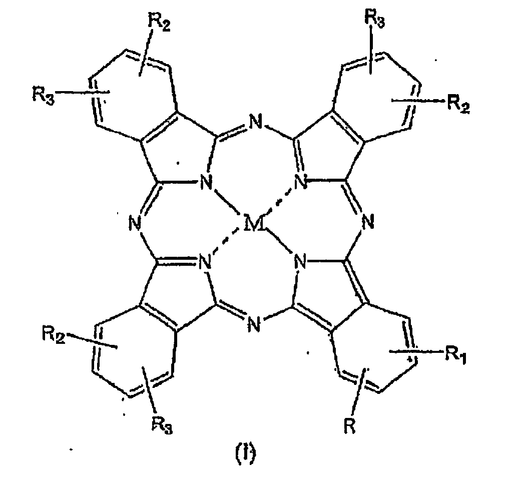 in which M is Zn,
R is selected from H, groups comprising at least one quaternary ammonium substituent, groups comprising at least one aliphatic amino substituent, and groups suitable for covalently binding to bio-organic carriers chosen in the group consisting of amino acids, polypeptides, proteins, antibodies, polysaccharides and aptamers,
R₁, equal or different from R₁ is selected from H, groups comprising at least one aliphatic amino substituent and groups comprising at least one quaternary ammonium substituent,
R₂ and R₃, equal or different from one another, are selected from H, alkoxy groups having-from 1 to 10 carbon atoms, -thioalkoxy groups having from 1 to 10 carbon atoms, groups comprising at least one aliphatic amino substituent and groups comprising at least one quaternary ammonium substituent, with the proviso that:
a) at least one amongst R, R_{1,} R₂ and R₃ is a group comprising at least one aliphatic amino substituent or a group comprising at least one quaternary ammonium substituent and, when R, R₁, R₂ and R₃ are groups comprising at least one aliphatic amino substituent or groups comprising at least one quaternary ammonium substituent, or R and R₂ are groups comprising at least one aliphatic amino substituent or groups comprising at least one quaternary ammonium substituent and R₁ and R₃ are H, said groups comprising at least one aliphatic amino substituent or said groups comprising at least one quaternary ammonium substituent, are the same;
b) when R and R₁ are both different from H, they are in positions 1,4 or 2,3, whereas when only one between R and R₁ is different from H, it is in position 1 or 2;
c) when R₂ and R₃ are both different from H, they are in positions 8, 11, 15, 18, 22, 25 or 9, 10, 16, 17, 23, 24 whereas when only one between R₂ and R₃ is different from H, it is in positions 8(11), 15(18), 22(25) or in positions 9(10), 16(17), 23(24); and their pharmaceutically acceptable salts,
for the preparation of pharmaceutical compositions for non-photodynamic treatment of microbial infections and treatment of diseases **characterised by** cell hyperproliferation.

2. Use according to claim 1, wherein said group comprising at least one quaternary ammonium substituent or one aliphatic amino substituent is a (X)ₚR₄ group in which X is selected from the group consisting of O -CH₂-, CO, S, SO, and - NR₅ where R₅ is chosen from H and C₁-C₁₅ alkyl; and R₄ is 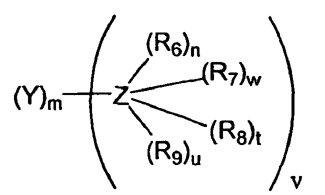 in which
Y is selected from the group consisting of C₁-C₁₀ alkyl and phenyl, possibly substituted, or Y forms with the Z group, to which it is bound, a saturated or unsaturated heterocycle, optionally substituted, which may comprise up to 2 heteroatoms selected from the group consisting of N, O and S;
Z is selected from the group consisting of -N, -CH₂N and -CONHCH₂CH₂N;
R₆ and R₇, equal or different from one another, are selected from the group consisting of C₁-C₁₅ alkyl and phenyl or form with the Z group, to which they are bound, a saturated or unsaturated heterocycle, optionally substituted, which may comprise up to two heteroatoms selected from the group consisting of N, O and S;
R₈ and R₉, equal or different from each other, are selected from the group consisting of H, C₁-C₁₅ alkyl, and R₁₀COOEt or R₁₀COOMe groups in which R₁₀ is C₁-C₁₅ alkyl;
m, n, p, w, t and u, independently from one another, are 0 or 1; and
v is an integer between 1 and 3,
with the proviso that only one amongst n, w, t and u is simultaneously 0.

3. Use according to claim 1, wherein said group comprising at least one quaternary ammonium substituent is selected from the following groups:

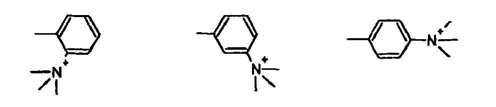

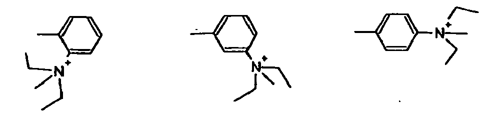

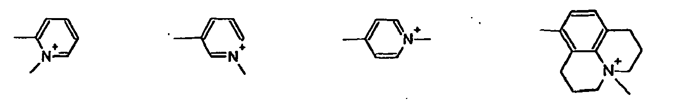

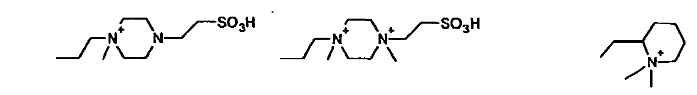

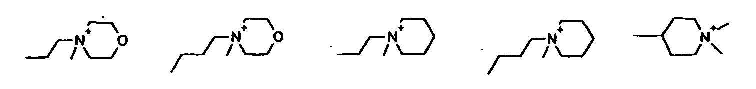

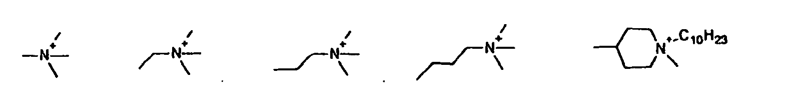

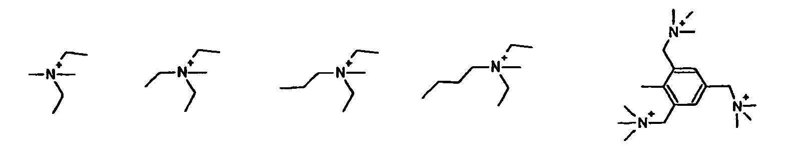

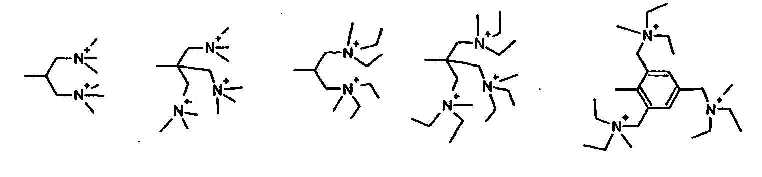

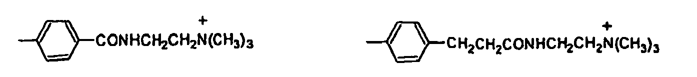

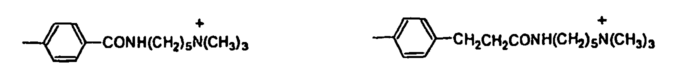

4. Use according to claim 1, wherein said group comprising at least one quaternary ammonium substituent is selected from the following groups:

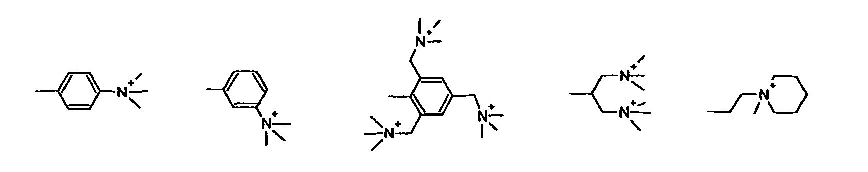

5. Use according to claim 1, wherein said group comprising at least one aliphatic amino substituent is selected from the following groups:

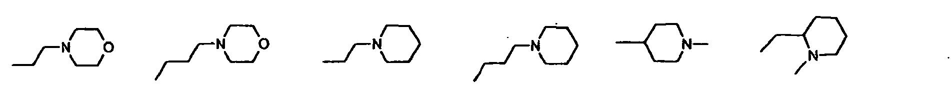

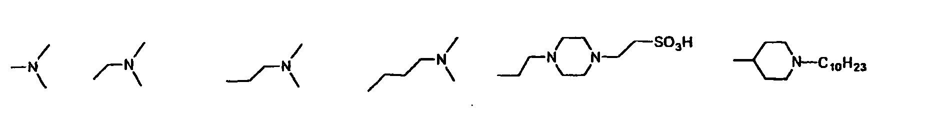

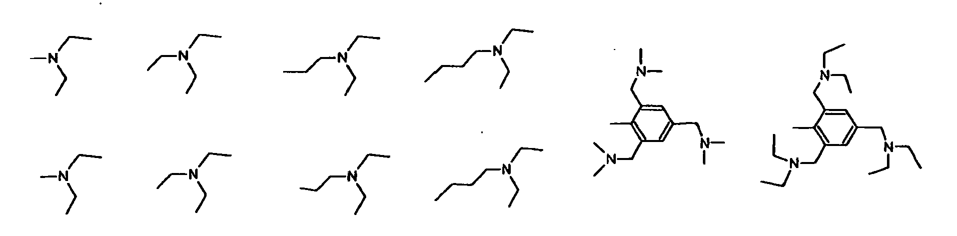

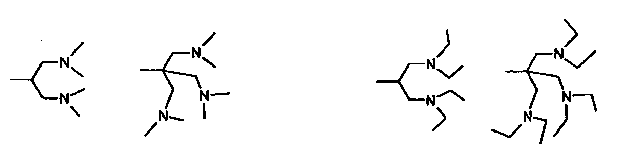

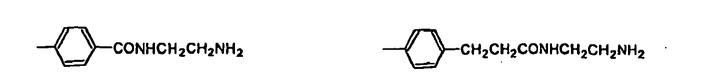

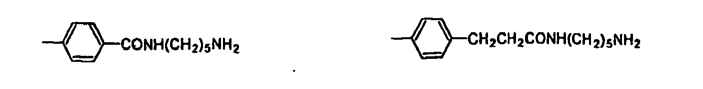

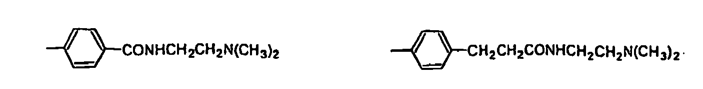

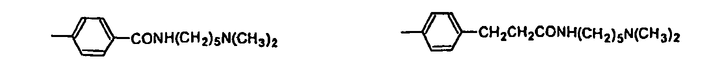

6. Use according to claim 1, wherein said group comprising at least one aliphatic amino substituent is selected from the following groups:

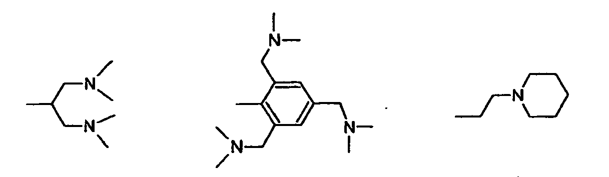

7. Use according to claim 1, wherein in said compounds of formula (I) M is Zn.

8. Use according to claim 1, wherein said saturated or unsaturated heterocycle is selected from the group consisting of morpholine, piperidine, pyridine, pyrimidine, piperazine, pyrrolidine, pyrroline, imidazole, aniline and julolidine.

9. Use according to claim 1, wherein said group suitable for conjugation to specific carriers is a group suitable for covalently binding to amino acids, polypeptides, proteins, antibodies, polysaccharides and aptamers.

10. Use according to claim 9, wherein said group suitable for conjugation to specific carriers is a group selected from the group consisting of -COOH, -SH, - NH₂, -CO-CH₂-Br, -SO₂Cl, maleimide, hydrazine, phenol, imidate, biotin, possibly bound to the phthalocyanine nucleus through a suitable spacer (X)ₚ-W, where X and p are as defined in claim 1 and W is chosen from C₁-C₁₀ alkyl, aryl, and C₁-C₅ arylalkyl.

11. Use according to claim 1, wherein R is a group suitable for conjugation to specific carriers, R₁ is equal to H and R₂ and R₃ are selected from H, groups comprising at least one aliphatic amino substituent, and groups comprising at least one quaternary ammonium substituent, provided that at least one of R₂ and R₃ is different from H.

12. Use according to claim 1, wherein said compounds of formula (I) are selected from:
compounds tetra-substituted with the same substituents, i.e. compounds in which the R and R₂ substituents are the same and selected from groups comprising at least one quaternary ammonium substituent and groups comprising at least one aliphatic amino substituent, as defined in the preceding claims, and R₁ = R₃ = H;
the tetra-substituted compounds of formula (I) suitable for conjugation, in which R is selected from groups suitable for conjugation to specific carriers, as defined in the preceding claims, R₂ is selected from groups comprising at least one quaternary ammonium substituent and groups comprising at least one aliphatic amino substituent, as defined in the preceding claims, and R₁ = R₃ = H; and
di- and mono-substituted compounds i.e. compounds in which: R is selected from groups comprising at least one quaternary ammonium substituent and groups comprising at least one aliphatic amino substituent, as defined in the preceding claims, R₁ = R or R₁ is H, and R₂ = R₃ = H.

13. Use according to claim 12, wherein said compounds of formula (I) are selected from compounds tetra-substituted with the same substituents, and di- and mono-substituted compounds as defined in claim 12.

14. Use according to claim 12, wherein said compounds of formula (I) are selected from di- and mono-substituted compounds as defined in claim 12.

15. Use according to claim 1, wherein said compounds of formula (I) are selected from the following compounds:
- compound of formula (I) in which M is Zn, R₁=R₂=R₃=H and R = {2-(trimethylammonium)-1-[(trimethylammonium) methyl]ethoxy} dichloride in position 2 [Compound 1],
- compound of general formula (I) in which M is Zn, R₁=R₂=R₃=H and R= {2-(dimethylamino)-1-[(dimethylamino) methyl]ethoxy} in position 2 [Compound 2],
- compound of general formula (I) in which M is Zn, R₁=R₂=R₃=H and R= {2-(trimethylammonium)-1-[(trimethylammonium) methyl]ethoxy} diiodide in position 1 [Compound 3],
- compound of general formula (I) in which M is Zn, R₁=R₂=R₃=H and R= [2-(N-methyl-1-piperidinium)ethoxy] iodide in position 1 [Compound 4],
- compound of general formula (I) in which M is Zn, R₁=R₂=R₃=H and R= [3-(N,N,N-trimethylammonium) phenoxy] iodide in position 2 [Compound 5],
- compound of general formula (I) in which M is Zn, R₁=R₂=R₃=H and R= [4-(N,N,N-trimethylammonium) phenoxy] iodide in position 1 [Compound 6],
- compound of general formula (I) in which M is Zn, R₁=R₂=R₃=H and R= (N-methylpyridinium-3-oxy) iodide in position 1 [Compound 7],
- compound of general formula (I) in which M is Zn, R₁=R₂=R₃=H and R= {2,4,6-tris-[(N,N- dimethylamino)methyl] phenoxy} in position 2 [Compound 8],
- compound of general formula (I) in which M is Zn, R₁=R₂=R₃=H and R= {2,4,6-tris-[(N,N,N-trimethylammonium)methyl]phenoxy} trichloride in position 2 [Compound 9],
- compound of general formula (I) in which M is Zn, R₂=R₃=H, R=R₁= {[3-(dimethylamino)phenyl]thio}, with R and R₁ in positions 2 and 3 [Compound 10],
- compound of general formula (I) in which M is Zn, R₂=R₃=H, R=R₁= {3-(*N,N,N-*trimethylammonium)phenoxy] iodide, with R and R₁ in positions 2 and 3 [Compound 11],
- compound of general formula (I) in which M is Zn, R₁=R₃=H, R=R₂= [3-(*N,N,N-*trimethylammonium)phenoxy] chloride, with R and R₂ in positions 2,9(10),16(17),23(24) [Compound 12],
- compound of general formula (I) in which M is Zn, R₁=R₃=H, R=R₂= [3-(*N,N,N-*trimethylammonium)phenoxy] chloride, with R and R₂ in positions 1, 8(11), 15(18), 22(25) [Compound 13],
- compound of general formula (I) in which M is Zn, R₁=R₃=H, R=R₂= [4-(*N,N,N-*trimethylammonium)phenoxy] chloride, with R and R₂ in positions 1, 8(11), 15(18), 22(25) [Compound 14], and
- compound of general formula (I) in which M is Zn, R=R₁=R₂= R₃= [3-(*N,N,N-*trimethylammonium)phenoxy] chloride, with R, R₁, R₂, R₃ in positions 2,3,9,10,16,17,23,24 [Compound 15]

16. Use according to claim 1, wherein said diseases **characterised by** cell hyperproliferation are selected from tumours, psoriasis, actinic keratosis, atheroma, endoarterial hyperplasia and prostatic hyperplasia.

17. Use according to claim 1, wherein said pharmaceutical compositions are useful in the treatment of microbial infections caused by Gram- bacteria, Gram+ bacteria, and fungi.

18. Use according to claim 1, wherein said pharmaceutical compositions are useful in the treatment of microbial infections caused by Gram+ bacteria and fungi.

19. Use according to claim 1, wherein said pharmaceutical compositions are useful in the treatment of microbial infections caused by fungi.

20. Use according to claim 1, wherein said pharmaceutical compositions comprise, in addition to the compounds of formula (I), one or more other active principles and/or a metal chelating agent, optionally in combination with one or more excipients and pharmaceutically acceptable diluents.

21. Use according to claim 20, wherein said metal chelating agent is selected from the group consisting of 1,2-diaminocyclohexane-N,N,N',N'-tetraacetic acid (CDTA), diethylenetriaminepentaacetic acid (DTPA), ethylenediamine-N,N,N',N'-tetraacetic acid (EDTA), citric acid and its salts.

22. Use according to claim 21, wherein said metal chelating agent is ethylenediamine-N,N,N',N'-tetraacetic acid (EDTA).

## Patentansprüche

1. Verwendung von Phthalocyanin-Derivaten mit der allgemeinen Formel (I), 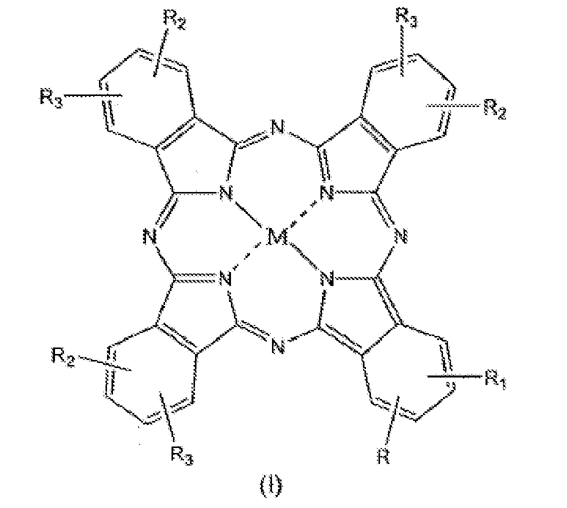 in welcher M für Zn steht,
R unter H ausgewählt ist sowie Gruppen, welche mindestens einen quaternären Ammoniumsubstituenten umfassen, Gruppen, die mindestens einen aliphatischen Aminosubstituenten umfassen, und Gruppen, die zur Bindung an bioorganische Trägerstoffe geeignet sind, welche aus der Gruppe ausgewählt sind, die aus Aminosäuren, Polypeptiden, Proteinen, Antikörpern, Polysacchariden und Aptameren besteht,
R1 gleich oder verschieden zu R ist und unter H ausgewählt ist sowie Gruppen, die mindestens einen aliphatischen Aminosubstituenten umfassen und Gruppen, die mindestens einen quaternären Aminosubstituenten umfassen,
R₂ und R₃ gleich oder verschieden voneinander sind, unter H ausgewählt sind sowie Alkoxy-Gruppen, welche 1 bis 10 Kohlenstoffatome enthalten, Thioalkoxygruppen, welche 1 bis 10 Kohlenstoffatome enthalten, Gruppen, die mindestens einen aliphatischen Aminosubstituenten umfassen, und Gruppen, die mindestens einen quaternären Ammoniumsubstituenten umfassen, unter der Bedingung, dass:
a) mindestens ein Rest unter R, R₁, R₂ und R₃ eine Gruppe darstellt, welche mindestens einen aliphatischen Aminosubstituenten umfasst oder eine Gruppe, welche mindestens einen quaternären Ammoniumsubstituenten umfasst und, wenn R, R₁, R₂ und R₃ Gruppen darstellen, welche mindestens einen aliphatischen Aminosubstituenten umfassen, oder Gruppen, welche mindestens einen quaternären Ammoniumsubstituenten umfassen, oder R und R₂ Gruppen darstellen, welche mindestens einen aliphatischen Aminosubstituenten umfassen, oder Gruppen, die mindestens einen quaternären Ammoniumsubstituenten umfassen, und R₁ und R₃ H darstellen, wobei die genannten Gruppen mindestens einen aliphatischen Aminosubstituenten umfassen, oder die genannten Gruppen mindestens einen quaternären Ammoniumsubstituenten umfassen, gleich sind;
b) wenn R und R₁ anders als H sind, sich an den Positionen 1,4 oder 2,3 befinden, wohingegen, wenn nur R oder R₁ anders als H ist, sich dieser Rest an Position 1 oder 2 befindet;
c) wenn sowohl R₂ als auch R₃ anders als H sind, sie sich an den Positionen 8, 11, 15, 18, 22, 25 oder 9, 10, 16, 17, 23, 24 befinden, wohingegen wenn nur R₂ oder R₃ verschieden von H ist, sich dieser Rest an den Positionen 8(11), 15(18), 22(25) oder an den Positionen 9(10), 16(17), 23(24) befindet; und ihre pharmazeutisch anerkannten Salze für die Herstellung von pharmazeutischen Zusammensetzungen für die nichtphotodynamische Behandlung von mibrobiellen Infektionen und Behandlung von Krankheiten, die durch Zellhyperproliferation **gekennzeichnet** sind.

2. Verwendung gemäß Anspruch 1, wobei genannte Gruppe, welche mindestens einen quaternären Ammoniumsubstituenten oder einen aliphatischen Aminosubstituenten umfasst, eine (X)ₚR₄-Gruppe darstellt, in welcher X aus der Gruppe ausgewählt ist, die aus O, -CH₂-, CO, S, SO und -NR₅ besteht, wobei R₅ ausgewählt ist unter H und C₁-C₁₅-Alkyl; und R₄ ist 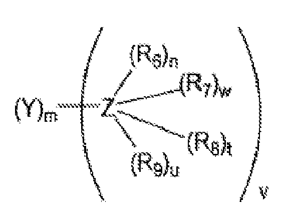 wobei
Y ausgewählt ist aus der Gruppe, welche aus C₁-C₁₀-Alkyl, und Phenyl besteht, möglicherweise substituiert, oder Y zusammen mit der Z-Gruppe, an welche es gebunden ist, einen gesättigten oder ungesättigten Heterozyklus bildet, welcher optional substituiert ist und bis zu 2 Heteroatome umfasst, ausgewählt aus der Gruppe bestehend aus N, O und S;
Z ausgewählt ist aus der Gruppe, welche aus -N, CH₂N und -CONHCH₂CH₂N besteht;
R₆ oder R₇ gleich oder verschieden voneinander sind, aus der Gruppe ausgewählt sind, welche aus C₁-C₁₅-Alkyl und Phenyl besteht, oder mit der Z-Gruppe, mit welcher sie verbunden sind, einen gesättigten oder ungesättigten Heterozyklus bilden, welcher optional substituiert ist und bis zu zwei Heteroatome umfasst, ausgewählt aus der Gruppe, bestehend aus N, O und S;
R₈ und R₉ gleich oder verschieden voneinander sind, aus der Gruppe ausgewählt sind, welche aus H, C₁-C₁₅-Alkyl besteht, sowie R₁₀COOEt- oder R₁₀COOMe-Gruppen, in welchen R₁₀für C₁-C₁₅-Alkyl steht;
m, n, p, w, t und u unabhängig voneinander 0 oder 1 darstellen; und v eine ganze Zahl zwischen 1 und 3 ist,
unter der Bedingung, dass nur einer unter n, w, t und u 0 ist.

3. Verwendung gemäß Anspruch 1, wobei genannte Gruppe, welche mindestens einen quaternären Ammoniumsubstituenten umfasst, ausgewählt ist aus den folgenden Gruppen:

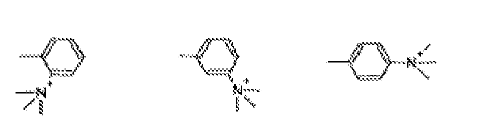

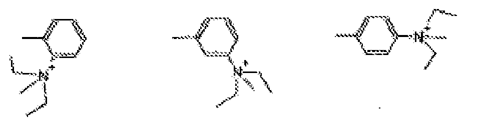

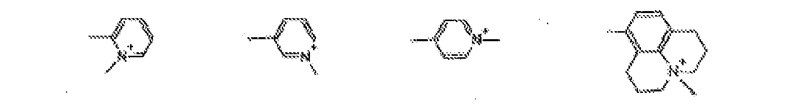

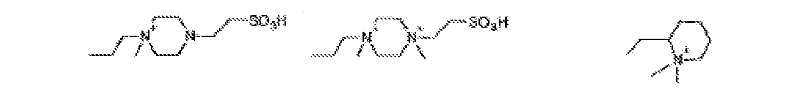

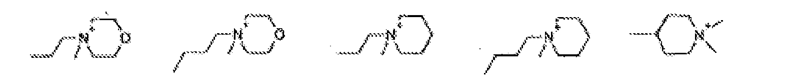

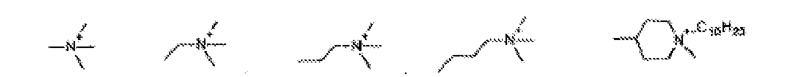

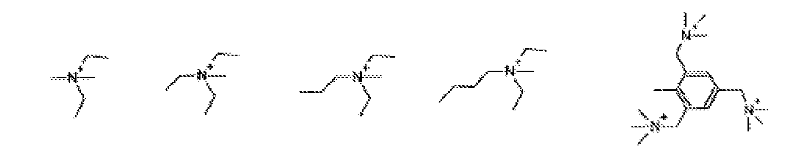

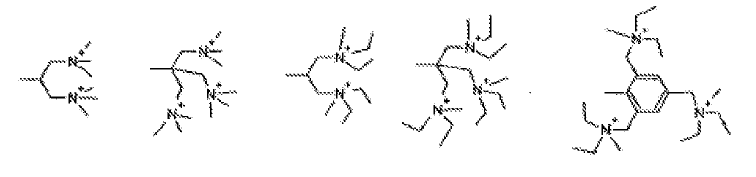

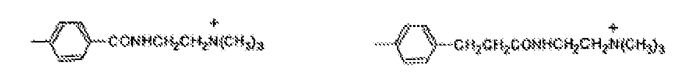

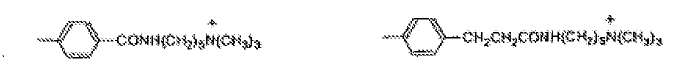

4. Verwendung gemäß Anspruch 1, wobei genannte Gruppe, welche mindestens einen quaternären Ammoniumsubstituenten umfasst, aus den folgenden Gruppen ausgewählt ist:

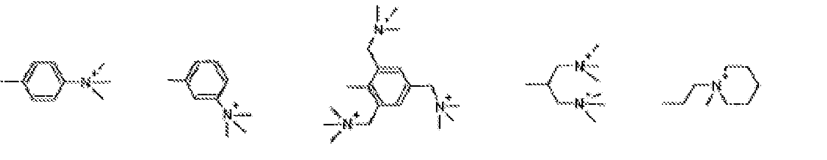

5. Verwendung gemäß Anspruch 1, wobei die genannte Gruppe, welche mindestens einen aliphatischen Aminosubstituenten umfasst, aus den folgenden Gruppen ausgewählt ist:

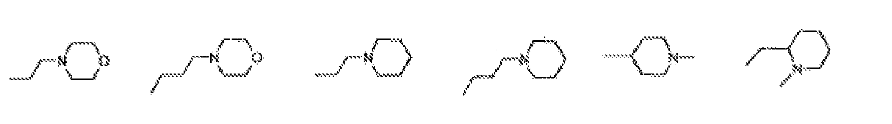

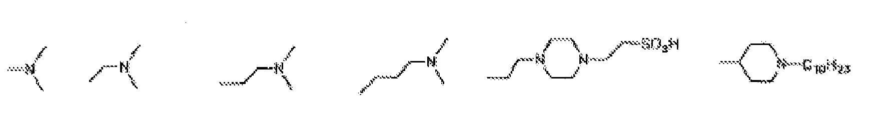

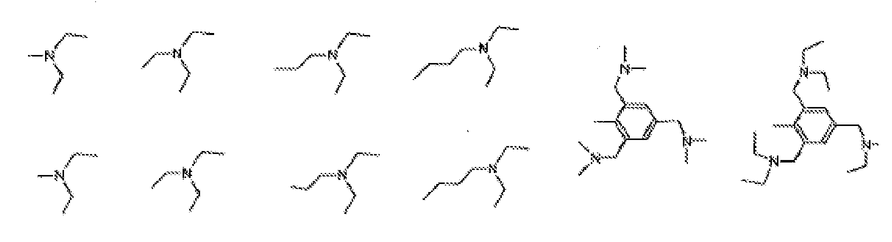

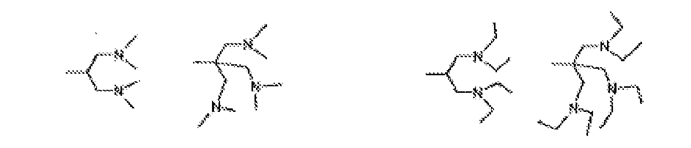

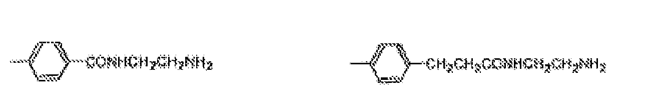

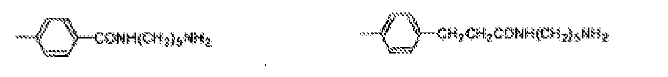

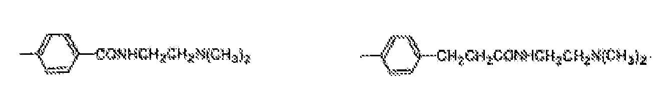

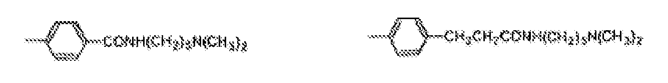

6. Verwendung gemäß Anspruch 1, wobei genannte Gruppe, welche mindestens einen aliphatischen Aminosubstituenten umfasst, aus den folgenden Gruppen ausgewählt ist:

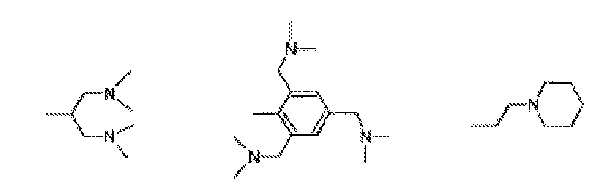

7. Verwendung gemäß Anspruch 1, wobei in den genannten Verbindungen mit der Formel (I) M für Z steht.

8. Verwendung gemäß Anspruch 1, wobei genannter gesättigter oder ungesättigter Heterozyklus aus der Gruppe ausgewählt ist, die aus Morpholin, Piperidin, Pyridin, Pyrimidin, Piperazin, Pyrrolidin, Pyrrolin, Imidazol, Anilin und Julolidin besteht.

9. Verwendung gemäß Anspruch 1, wobei es sich bei der genannten Gruppe, welche für die Konjugation mit speziellen Trägerstoffen geeignet ist, um eine Gruppe handelt, die sich für eine kovalente Bindung mit Aminosäuren, Polypeptiden, Proteinen, Antikörpern, Polysacchariden und Aptameren eignet.

10. Verwendung gemäß Anspruch 9, wobei es sich bei der genannten Gruppen, welche für die Konjugation mit speziellen Trägerstoffen geeignet ist, um eine Gruppe handelt, welche aus der Gruppe ausgewählt ist, bestehend aus -COOH, -SH, -NH₂, -CO-CH₂-Br, -SO₂Cl, Maleimid, Hydrazin, Phenol, Imidat, Biotin, möglicherweise gebunden an einen Phthalocyaninkern über einen geeigneten Spacer (X)ₚ-W, wobei X und p wie in Anspruch 1 definiert sind, und W ausgewählt ist unter C₁-C₁₀-Alkyl, Aryl und C₁-C₅-Arylakyl.

11. Verwendung gemäß Anspruch 1, wobei R eine Gruppe ist, die sich für die Konjugation mit spezifischen Trägerstoffen eignet, R₁ gleich H ist und R₂ und R₃ unter H ausgewählt sind, Gruppen, welche mindestens einen aliphatischen Aminosubstituenten umfassen, sowie Gruppen, welche mindestens einen quaternären Ammoniumsubstituenten umfassen, unter der Bedingung, dass mindestens R₂ oder R₃ anders als H ist.

12. Verwendung gemäß Anspruch 1, wobei genannte Verbindungen mit der Formel (I) ausgewählt sind unter:
Verbindungen, die mit den gleichen Substituenten tetra-substituiert sind, d. h. Verbindungen, in welchen die R- und R₂-Substituenten gleich sind und aus den Gruppen ausgewählt sind, welche mindestens einen quaternären Ammoniumsubstituenten umfassen, sowie Gruppen, die mindestens einen aliphatischen Aminosubstituenten umfassen, wie in den vorherigen Ansprüchen definiert, und R₁ = R₃ = H sind;
den tetra-substituierten Verbindungen mit der Formel (I), welche für die Konjugation geeignet sind, in welchen R aus den Gruppen ausgewählt ist, welche sich für die Konjugation mit spezifischen Trägerstoffen eignen, wie in der vorherigen Ansprüchen definiert, R₂ aus den Gruppen ausgewählt ist, welche mindestens einen quaternären Ammoniumsubstituenten umfassen, sowie Gruppen, welche mindestens einen aliphatischen Aminosubstituenten umfassen, wie in den vorherigen Ansprüchen definiert, und R₁ = R₃ = H sind; und
di- und mono-substituierten Verbindungen, d. h. Verbindungen, in welchen: R aus den Gruppen ausgewählt ist, welche mindesten einen quaternären
Ammoniumsubstituenten umfassen, sowie Gruppen, welche mindestens einen aliphatischen Aminosubstituenten umfassen, wie in den vorherigen Ansprüchen definiert, R₁ = R oder R₁ ist H und R₂ = R₃ = H.

13. Verwendung gemäß Anspruch 12, wobei genannte Verbindungen mit der Formel (I) ausgewählt sind unter Verbindungen, welche mit den gleichen Substituenten tetra-substituiert sind, sowie di- und mono-substituierten Verbindungen, wie in Anspruch 12 definiert.

14. Verwendung gemäß Anspruch 12, wobei genannte Verbindungen mit der Formel (I) aus di- und monosubstituierten Verbindungen, wie in Anspruch 12 definiert, ausgewählt sind.

15. Verwendung gemäß Anspruch 1, wobei genannte Verbindungen mit der Formel (I) unter den folgenden Verbindungen ausgewählt sind:
- Verbindung mit der Formel (I), in welcher M für Zn steht, R₁ = R₂ = R₃ = H und R = {2-(trimethylammonium)-1-[(trimethylammonium)methyl]ethoxy}dichlorid an Position 2 [Verbindung 1],
- Verbindung mit der allgemeinen Formel (I), in welcher M für Zn steht, R₁ = R₂ = R₃ = H und R = {2-(dimethylamino)-1-[(dimethylamino)methyl]ethoxy} an Position 2 [Verbindung 2],
- Verbindung mit der allgemeinen Formel (I), in welcher M für Zn steht, R₁ = R₂ = R₃ = H und R = {2-(trimethylammonium)-1[(trimethylammonium) methyl]ethoxy}diiodid an Position 1 [Verbindung 3]
- Verbindung mit der allgemeinen Formel (I), in welcher M für Zn steht, R₁ = R₂ = R₃ = H und R = [2-(N-methyl-lpiperidinium)ethoxy]iodid an Position 1 [Verbindung 4],
- Verbindung mit der allgemeinen Formel (I), in welcher M für Zn steht, R₁ = R₂ = R₃ = H und R = [3-(N,N,N-trimethylammonium)phenoxy]iodid an Position 2 [Verbindung 5],
- Verbindung mit der allgemeinen Formel (I), in welcher M für Zn steht, R₁ = R₂ = R₃ = H und R = [4-(N,N,N-trimethylammonium)phenoxy]iodid an Position 1 [Verbindung 6],
- Verbindung mit der allgemeinen Formel (I), in welcher M für Zn steht, R₁ = R₂ = R₃ = H und R = (N-methylpyridinium-3-oxy)iodid an Position 1 [Verbindung 7],
- Verbindung mit der allgemeinen Formel (I), in welcher M für Zn steht, R₁ = R₂ = R₃ = H und R = {2,4,6-tris-[(N,N-dimethylamino)methyl]phenoxy} an Position 2 [Verbindung 8],
- Verbindung mit der allgemeinen Formel (I), in welcher M für Zn steht, R₁ = R₂ = R₃ = H und R = {2,4,6-tris-[(N,N,N-trimethylammonium)methyl]phenoxy}trichlorid an Position 2 [Verbindung 9],
- Verbindung mit der allgemeinen Formel (I), in welcher M für Zn steht, R₂ = R₃ = H und R = R₁= {[3-(dimethylamino)phenyl]thio}, wobei R und R₁ sich an den Positionen 2 und 3 befinden [Verbindung 10],
- Verbindung mit der allgemeinen Formel (I), in welcher M für Zn steht, R₂ = R₃ = H und R = R₁= {3-(N,N,N-trimethylammonium)phenoxy]iodid, wobei R und R₁ sich an den Positionen 2 und 3 befinden [Verbindung 11],
- Verbindung mit der allgemeinen Formel (I), in welcher M für Zn steht, R₁ = R₃ = H und R = R₂= [3-(N,N,N-trimethylammonium)phenoxy]chlorid, wobei R und R₂ sich an den Positionen 2, 9(10), 16(17), 23(24) befinden [Verbindung 12],
- Verbindung mit der allgemeinen Formel (I), in welcher M für Zn steht, R₁ = R₃ = H und R = R₂= [3-N,N,N-trimethylammonium)phenoxy]chlorid, wobei R und R₂ sich an den Positionen 1, 8(11), 15(18), 22(25) befinden [Verbindung 13],
- Verbindung mit der allgemeinen Formel (I), in welcher M für Zn steht, R₁ = R₃ = H und R = R₂= [4-(N,N,N-trimethylammonium)phenoxy]chlorid, wobei R und R₂ sich an den Positionen 1, 8(11), 15(18), 22(25) befinden [Verbindung 14] und
- Verbindung mit der allgemeinen Formel (I), in welcher M für Zn steht, R = R₁ = R₂ = R₃ = [3-(N,N,N-trimethylammonium)phenoxy]chlorid, wobei R, R₁, R₂, R₃ sich an den Positionen 2, 3, 9, 10, 16, 17, 23, 24 befinden [Verbindung 15].

16. Verwendung gemäß Anspruch 1, wobei genannte Krankheiten **dadurch gekennzeichnet sind, dass** eine Zellhyperproliferation stattfindet und diese Krankheiten ausgewählt sind unter Tumorerkrankungen, Psoriasis, aktinischer Keratose, Atherom, endoarterieller Hyperplasie und Prostatahyperplasie.

17. Verwendung gemäß Anspruch 1, wobei genannte pharmazeutische Zusammensetzungen hilfreich bei der Behandlung mikrobieller Infektionen sind, die durch Gram⁻ Bakterien, Gram⁺ Bakterien und Pilze verursacht werden.

18. Verwendung gemäß Anspruch 1, wobei genannte pharmazeutische Zusammensetzungen hilfreich bei der Behandlung mikrobieller Infektionen sind, die durch Gram⁺ Bakterien und Pilze verursacht werden.

19. Verwendung gemäß Anspruch 1, wobei genannte pharmazeutische Zusammensetzungen hilfreich bei der Behandlung mikrobieller Infektionen sind, die durch Pilze verursacht werden.

20. Verwendung gemäß Anspruch 1, wobei genannte pharmazeutische Zusammensetzungen zusätzlich zu den Verbindungen mit der Formel (I) einen oder mehrere Wirkstoff(e) und/oder einen Metall-Chelatbildner, optional in Verbindung mit einem oder mehreren Hilfsstoff(en) und pharmazeutisch anerkannten Verdünnungsmitteln enthalten.

21. Verwendung gemäß Anspruch 20, wobei genannter Metall-Chelatbildner aus der Gruppe ausgewählt ist, welche aus 1,2-Diaminocyclohexan-N,N,N',N'-tetraessigsäure (CDTA), Diethylentriaminpentaessigsäure (DTPA), Ethylendiamin-N,N,N',N'-tetraessigsäure (EDTA), Zitronensäure und deren Salze besteht.

22. Verwendung gemäß Anspruch 21, wobei genannter Metall-Chelatbildner Ethylendiamin-N,N,N',N'-tetraessigsäure (EDTA) ist.

## Revendications

1. Utilisation de dérivés de phtalocyanine de formule générale (I) : 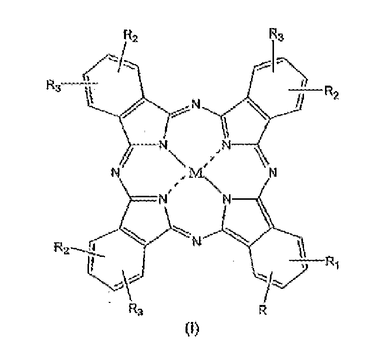 dans laquelle M est Zn,
R est choisi parmi H, des groupes comportant au moins un substituant ammonium quaternaire, des groupes comportant au moins un substituant amine aliphatique, et des groupes adaptés pour se lier par covalence à des vecteurs bio-organiques choisis dans le groupe constitué d'acides aminés, de polypeptides, de protéines, d'anticorps, de polysaccharides et d'aptamères,
R₁, identique à R ou différent de celui-ci, est choisi parmi H, des groupes comportant au moins un substituant amine aliphatique et des groupes comportant au moins un substituant ammonium quaternaire,
R₂ et R₃, identiques l'un à l'autre ou différents l'un de l'autre, sont choisis parmi H, des groupes alcoxy ayant de 1 à 10 atomes de carbone, des groupes thioalcoxy ayant de 1 à 10 atomes de carbone, des groupes comportant au moins un substituant amine aliphatique et des groupes comportant au moins un substituant ammonium quaternaire, à condition que :
a) au moins un élément parmi R, R₁, R₂ et R₃ soit un groupe comportant au moins un substituant amine aliphatique ou un groupe comportant au moins un substituant ammonium quaternaire et, lorsque R, R₁, R₂ et R₃ sont des groupes comportant au moins un substituant amine aliphatique ou des groupes comportant au moins un substituant ammonium quaternaire, ou que R et R₂ sont des groupes comportant au moins un substituant amine aliphatique ou des groupes comportant au moins un substituant ammonium quaternaire et que R₁ et R₃ sont H, alors lesdits groupes comportant au moins un substituant amine aliphatique ou lesdits groupes comportant au moins un substituant d'ammonium quaternaire, soient identiques,
b) lorsque R et R₁ sont tous les deux différents de H, ils soient en positions 1,4 ou 2,3, alors que lorsqu'un seul entre R et R₁ est différent de H, il soit en position 1 ou 2,
c) lorsque R₂ et R₃ sont tous les deux différents de H, ils soient en positions 8, 11, 15, 18, 22, 25 ou 9, 10, 16, 17, 23, 24 alors que lorsqu'un seul élément entre R₂ et R₃ est différent de H, il soit en positions 8(11), 15(18), 22(25) ou en positions 9(10), 16(17), 23(24),
et leurs sels pharmaceutiquement acceptables,
pour la préparation de compositions pharmaceutiques pour le traitement non-photodynamique d'infections microbiennes et le traitement de maladies **caractérisées par** une hyperprolifération cellulaire.

2. Utilisation selon la revendication 1, dans laquelle ledit groupe comportant au moins un substituant ammonium quaternaire ou un substituant amine aliphatique est un groupe (X)ₚR₄ dans lequel X est choisi dans le groupe constitué de 0, -CH₂-, CO, S, SO et -NR₅, où R₅ est choisi parmi H et alkyle en C₁ à C₁₅, et R₄ est : 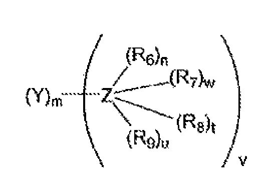 dans lequel
Y est choisi dans le groupe constitué d'un alkyle en C₁ à C₁₀ et de phényle, éventuellement substitué, ou Y forme avec le groupe Z, auquel il est lié, un hétérocycle saturé ou insaturé, éventuellement substitué, qui peut comporter jusqu'à 2 hétéroatomes choisis parmi le groupe constitué de N, 0 et S,
Z est choisi dans le groupe constitué de -N, -CH₂N et -CONHCH₂CH₂N,
R₆ et R₇, identiques ou différents l'un de l'autre, sont choisis dans le groupe constitué d'un alkyle en C₁ à C₁₅ et d'un phényle ou forment avec le groupe Z, auquel ils sont liés, un hétérocycle saturé ou insaturé, éventuellement substitué, qui peut comporter jusqu'à deux hétéroatomes choisis dans le groupe constitué de N, 0 et S,
R₈ et R₉, identiques ou différents l'un de l'autre, sont choisis dans le groupe constitué de H, alkyle en C₁ à C₁₅, et des groupes R₁₀COOEt ou R₁₀COOMe dans lesquels R₁₀ est un alkyle en C₁ à C₁₅,
m, n, p, w, t et u, indépendamment les uns des autres, sont égaux à 0 ou 1, et
v est un entier compris entre 1 et 3,
à condition qu'un seul élément parmi n, w, t et u soit simultanément égal à 0.

3. Utilisation selon la revendication 1, dans laquelle ledit groupe comportant au moins un substituant ammonium quaternaire est choisi parmi les groupes suivants :

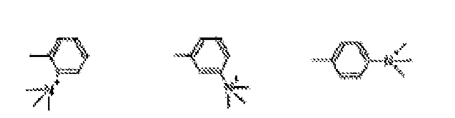

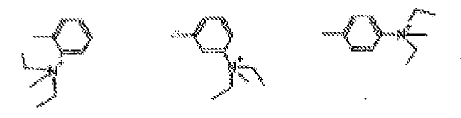

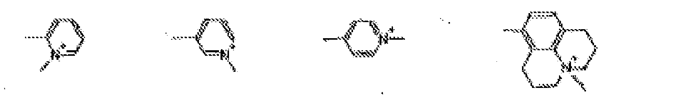

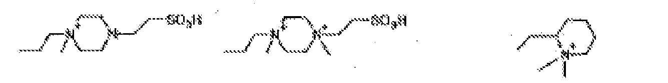

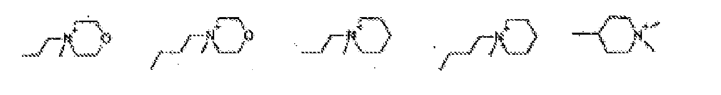

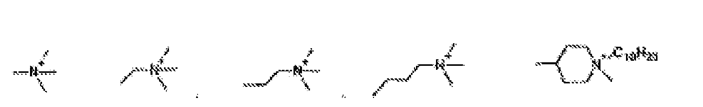

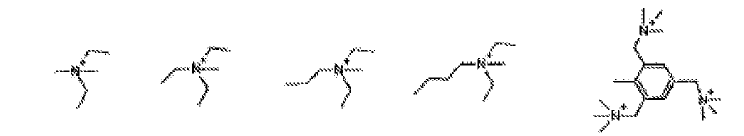

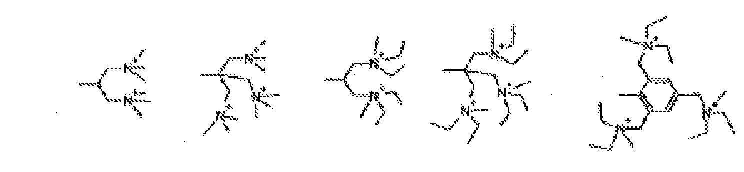

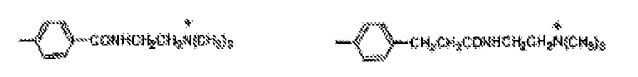

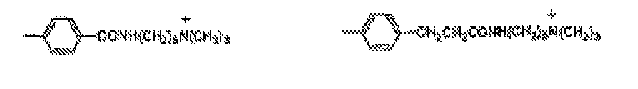

4. Utilisation selon la revendication 1, dans laquelle ledit groupe comportant au moins un substituant ammonium quaternaire est choisi parmi les groupes suivants :

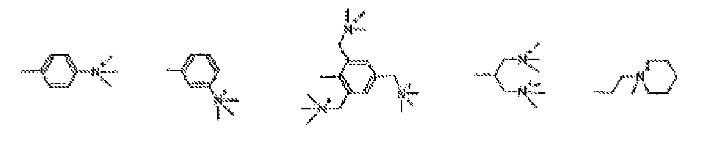

5. Utilisation selon la revendication 1, dans laquelle ledit groupe comportant au moins un substituant amine aliphatique est choisi parmi les groupes suivants :

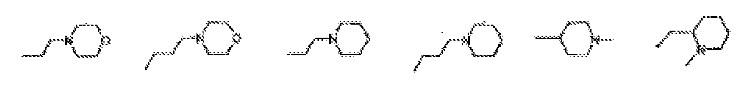

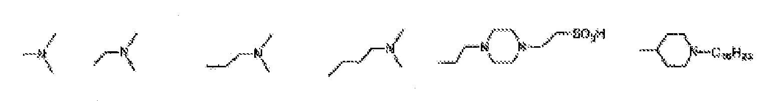

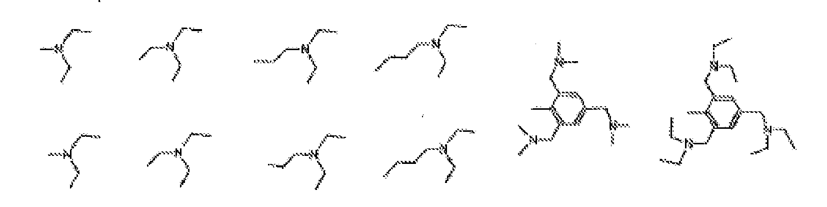

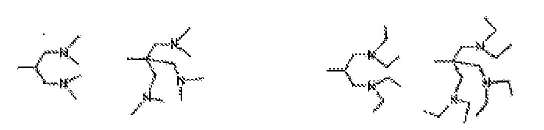

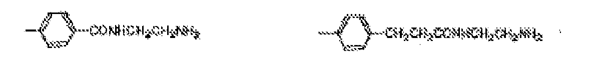

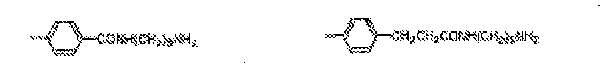

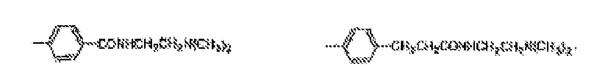

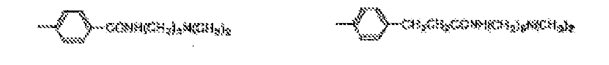

6. Utilisation selon la revendication 1, dans laquelle ledit groupe comportant au moins un substituant amine aliphatique est choisi parmi les groupes suivants :

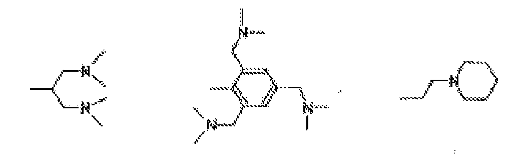

7. Utilisation selon la revendication 1, dans laquelle, dans lesdits composés de formule (I), M représente Zn.

8. Utilisation selon la revendication 1, dans laquelle ledit hétérocycle saturé ou insaturé est choisi dans le groupe constitué de morpholine, de pipéridine, de pyridine, de pyrimidine, de pipérazine, de pyrrolidine, de pyrroline, d'imidazole, d'aniline et de julolidine.

9. Utilisation selon la revendication 1, dans laquelle ledit groupe adapté pour une conjugaison à des vecteurs spécifiques est un groupe adapté pour se lier par covalence à des acides aminés, des polypeptides, des protéines, des anticorps, des polysaccharides et des aptamères.

10. Utilisation selon la revendication 9, dans laquelle ledit groupe adapté pour une conjugaison à des vecteurs spécifiques est un groupe choisi dans le groupe constitué de -COOH, -SH, -NH₂, -CO-CH₂-Br, -SO₂Cl, du maléimide, de l'hydrazine, du phénol, de l'imidate, de la biotine, éventuellement lié au noyau de phtalocyanine par un espaceur (X)ₚ-W adapté, où X et p sont tels que définis dans la revendication 1 et W est choisi parmi alkyle en C₁ à C₁₀, aryle et arylalkyle en C₁ à C₅.

11. Utilisation selon la revendication 1, dans laquelle R est un groupe adapté pour une conjugaison à des vecteurs spécifiques, R₁ est égal à H et R₂ et R₃ sont choisis parmi H, des groupes comportant au moins un substituant amine aliphatique, et des groupes comportant au moins un substituant ammonium quaternaire, à condition qu'au moins un élément parmi R₂ et R₃ soit différent de H.

12. Utilisation selon la revendication 1, dans laquelle lesdits composés de formule (I) sont choisis parmi :
des composés tétra-substitués avec les mêmes substituants, c'est-à-dire des composés dans lesquels les substituants R et R₂ sont identiques et sont choisis parmi des groupes comportant au moins un substituant ammonium quaternaire et des groupes comportant au moins un substituant amine aliphatique, tels que définis dans les revendications précédentes, et R₁ = R₃ = H,
les composés tétra-substitués de formule (I) adaptés pour une conjugaison, dans lesquels R est choisi parmi des groupes adaptés pour une conjugaison à des vecteurs spécifiques, tels que définis dans les revendications précédentes, R₂ est choisi parmi des groupes comportant au moins un substituant ammonium quaternaire et des groupes comportant au moins un substituant amine aliphatique, tels que définis dans les revendications précédentes, et R₁ = R₃ = H, et
des composés di- et mono-substitués, c'est-à-dire des composés dans lesquels : R est choisi parmi des groupes comportant au moins un substituant ammonium quaternaire et des groupes comportant au moins un substituant amine aliphatique, tels que définis dans les revendications précédentes, R₁ = R ou R₁ est H, et R₂ = R₃ = H.

13. Utilisation selon la revendication 12, dans laquelle lesdits composés de formule (I) sont choisis parmi des composés tétra-substitués avec les mêmes substituants, et des composés di- et mono-substitués tels que définis dans la revendication 12.

14. Utilisation selon la revendication 12, dans laquelle lesdits composés de formule (I) sont choisis parmi des composés di- et mono-substitués tels que définis dans la revendication 12.

15. Utilisation selon la revendication 1, dans laquelle lesdits composés de formule (I) sont choisis parmi les composés suivants :
- composé de formule (I) dans laquelle M est Zn, R₁ = R₂ = R₃ = H et R = dichlorure de {2-(triméthylammonium)-1-[(triméthylammonium)méthyl]éthoxyl en position 2 [Composé 1],
- composé de formule générale (I) dans laquelle M est Zn, R₁ = R₂ = R₃ = H et R = {2-(diméthylamino)-1-[{diméthylamino)méthyl]éthoxyl en position 2 [Composé 2],
- composé de formule générale (I) dans laquelle M est Zn, R₁ = R₂ = R₃ = H et R = diiodure de {2-(triméthylammonium)-1-[(triméthylammonium)méthyl]éthoxy} en position 1 [Composé 3],
- composé de formule générale (I) dans laquelle M est Zn, R₁ = R₂ = R₃ = H et R = iodure de [2-(N-méthyl-1-pipéridinium)éthoxy] en position 1 [Composé 4],
- composé de formule générale (I) dans laquelle M est Zn, R₁ = R₂ = R₃ = H et R = iodure de [3-(N,N,N-triméthylammonium)phénoxy] en position 2 [Composé 5],
- composé de formule générale (I) dans laquelle M est Zn, R₁ = R₂ = R₃ = H et R = iodure de [4-(N,N,N-triméthylammonium)phénoxy] en position 1 [Composé 6],
- composé de formule générale (I) dans laquelle M est Zn, R₁ = R₂ = R₃ = H et R = iodure de (N-méthylpyridinium-3-oxy) en position 1 [Composé 7],
- composé de formule générale (I) dans laquelle M est Zn, R₁ = R₂ = R₃ = H et R = {2,4,6-tris-[([N,N-diméthylamino)méthyl]phénoxy} en position 2 [Composé 8],
- composé de formule générale (I) dans laquelle M est Zn, R₁ = R₂ = R₃ = H et R = trichlorure de {2,4,6-tris-[(N,N,N-triméthylammonium)méthyl]phénoxy} en position 2 [Composé 9],
- composé de formule générale (I) dans laquelle M est Zn, R₂ = R₃ = H et R = R₁ = {[3-(diméthylamino)phényl]thio}, avec R et R₁ en positions 2 et 3 [Composé 10],
- composé de formule générale (I) dans laquelle M est Zn, R₂ = R₃ = H, R = R₁ = iodure de {3-(N,N,N-triméthylammonium)phénoxy], avec R et R₁ en positions 2 et 3 [Composé 11],
- composé de formule générale (I) dans laquelle M est Zn, R₁ = R₃ = H, R = R₂ = chlorure de [3-(N,N,N-triméthylammonium)phénoxy], avec R et R₂ en positions 2, 9 (10), 16 (17), 23 (24) [Composé 12],
- composé de formule générale (I) dans laquelle M est Zn, R₁ = R₃ = H, R = R₂ = chlorure de [3-(N,N,N-triméthylammonium)phénoxy], avec R et R₂ en positions 1, 8 (11), 15 (18), 22 (25) [Composé 13],
- composé de formule générale (I) dans laquelle M est Zn, R₁ = R₃ = H, R = R₂ = chlorure de [4-(N,N,N-triméthylammonium)phénoxy], avec R et R₂ en positions 1, 8 (11), 15 (18), 22 (25) [Composé 14], et
- composé de formule générale (I) dans laquelle M est Zn, R = R₁ = R₂ = R₃ = chlorure de [3-(N,N,N-triméthylammonium)phénoxy], avec R, R₁, R₂, R₃ en positions 2, 3, 9, 10, 16, 17, 23, 24 [Composé 15].

16. Utilisation selon la revendication 1, dans laquelle lesdites maladies **caractérisées par** une hyperprolifération cellulaire sont choisies parmi des tumeurs, le psoriasis, la kératose actinique, l'athérome, l'hyperplasie endoartérielle et l'hyperplasie prostatique.

17. Utilisation selon la revendication 1, dans laquelle lesdites compositions pharmaceutiques sont utiles dans le traitement d'infections microbiennes dues aux bactéries Gram-, aux bactéries Gram+ et à des champignons.

18. Utilisation selon la revendication 1, dans laquelle lesdites compositions pharmaceutiques sont utiles dans le traitement d'infections microbiennes dues aux bactéries Gram+ et à des champignons.

19. Utilisation selon la revendication 1, dans laquelle lesdites compositions pharmaceutiques sont utiles dans le traitement d'infections microbiennes dues à des champignons.

20. Utilisation selon la revendication 1, dans laquelle lesdites compositions pharmaceutiques comportent, en plus des composés de formule (I), un ou plusieurs autres principes actifs et/ou un agent chélateur de métaux, éventuellement en combinaison avec un ou plusieurs excipients et diluants pharmaceutiquement acceptables.

21. Utilisation selon la revendication 20, dans laquelle ledit agent chélateur de métaux est choisi dans le groupe constitué de l'acide 1,2-diaminocyclohexane-N,N,N',N'-tétra-acétique (CDTA), de l'acide diéthylènetriaminepenta-acétique (DTPA), de l'acide éthylènediamine-N,N,N',N'-tétra-acétique (EDTA), de l'acide citrique et de ses sels.

22. Utilisation selon la revendication 21, dans laquelle ledit agent chélateur de métaux est l'acide éthylènediamine-N,N,N',N'-tétraacétique (EDTA).
